# EUROPÄISCHE PATENTSCHRIFT

(11) **EP 3 439 718 B1**
(45) Veröffentlichungstag und Bekanntmachung des Hinweises auf die Patenterteilung: **11.09.2019**
(21) Anmeldenummer: 16787862.8
(22) Anmeldetag: 27.10.2016
(51) Int. Cl.: A61M 15/00, A61M 15/02, A61G 10/02, B02C 17/00, B02C 15/10, A47J 42/06, B02C 17/10

(54) **MIKRONISATOR**
MICRONISATION MEANS
DISPOSITIF DE MICRONISATION

(30) Priorität: 07.04.2016 DE 102016106358
(43) Veröffentlichungstag der Anmeldung: 13.02.2019
(73) Patentinhaber: Klafs GmbH & Co. KG, 74523 Schwaebisch Hall (DE)
(72) Erfinder: GÄBELE, Markus, 74523 Schwäbisch Hall (DE)
(74) Vertreter: Witte, Weller & Partner Patentanwälte mbB
(86) Internationale Anmeldenummer: PCT/EP2016/075904
(87) Internationale Veröffentlichungsnummer: WO 2017/174164

(56) Entgegenhaltungen:
- WO-A1-2007/054604
- WO-A1-2011/100981
- WO-A1-2015/006838
- WO-A2-2008/084269
- DE-C- 81 500
- US-A- 1 894 106
- US-A1- 2013 125 888

## Beschreibung

Die vorliegende Erfindung betrifft eine Vorrichtung zum Mikronisieren eines anorganischen Salzes. Eine derartige Vorrichtung wird häufig auch als Mikronisator bezeichnet. Sie dient der Herstellung von Salzpartikeln im Mikrometer- sowie Submikrometerbereich zur Erzeugung eines Salz-Schwebstaubs bzw. eines Salzaerosols.

Solche Mikronisatoren werden üblicherweise in Salzkammern eingesetzt, in welchen das Salzaerosol eingeatmet werden kann. Dies kann zu medizinischen oder kosmetischen Zwecken erfolgen, aber auch zu reinen Wohlfühlzwecken. Vermehrt werden Mikronisatoren auch in Saunen oder anderen Wellnessanwendungen zu oben genannten Zwecken eingesetzt.

Um in derartigen Anwendungen als Aerosol effektiv zu sein, sollten die im Aerosol vorhandenen Salzpartikel einen mittleren Teilchendurchmesser von etwa 2 µm, vorzugsweise von weniger als 2 µm aufweisen, so dass diese möglichst tief in die Lungenwege eindringen können.

Zur Erzeugung von mikronisierten Salzpartikeln mit mittleren Teilchendurchmessern im Bereich der oben genannten Werte existieren bereits verschiedene Technologien.

So beschreibt zum Beispiel die DE 10 2004 059 530 A1 eine Vorrichtung zum Herstellen eines Salzaerosols, bei der der Mahlvorgang durch ein schnell rotierendes Mahlwerkzeug erfolgt. Die in diesem Dokument beschriebene Vorrichtung hat allerdings den Nachteil, dass hier ein Mahlen mit einer mechanischen Mahlvorrichtung erfolgt, was zum einen im Hinblick auf die Abnutzung der Mahlvorrichtung sowie auf den Eintrag von Verunreinigungen, sei es aus dem Mahlwerkzeug oder durch Schmierstoffe aus dem Antrieb nachteilig ist. Ferner hat sich auch gezeigt, dass solche Vorrichtungen aufgrund der relativ häufigen Wartungsintervalle, die unter anderem auch auf die Verarbeitung von korrosivem Kochsalz zurückzuführen sind, für den kontinuierlichen Betrieb nur begrenzt geeignet sind. Abschließend haben diese Vorrichtungen noch den Nachteil, dass diese zu einer relativ hohen Geräuschentwicklung führen, so dass eine derartige Vorrichtung bei einem Einsatz in einer Wellnessanwendung eher als störend wahrgenommen wird.

Die US 5,747,002 beschreibt eine Vorrichtung zur Herstellung eines Salzaeorols unter Verwendung eines Strahlmühlenprinzips. Bei dieser Vorrichtung erfolgt das Mahlen im Gegenstromprinzip gefolgt von einer Sichtung basierend auf einem Zyklon. Auch wenn Strahlmühlen im Allgemeinen einige der oben beschriebenen Nachteile nicht aufweisen, weist die in der US 5,747,002 A gezeigte Vorrichtung zum einen den Nachteil auf, dass deren Konstruktion relativ komplex ist und zum anderen, dass dort Gase unter hohem Druck verwendet werden, was zu einem hohen Energieverbrauch sowie zu einem Sicherheitsrisiko führt. Ferner lassen sich mit dieser Vorrichtung auch lediglich Partikel mit einem Durchmesser von etwa 7 µm herstellen.

Die EP 2 457 559 A1 beschreibt eine weitere Vorrichtung zur Herstellung eines Salzaerosols. Das zu mikronisierende Salz liegt dabei in einer Rückhaltestufe vor und wird einem Mahlvorgang unterzogen. Eine Trennstufe ist mit der Rückhaltestufe, der Mahlstufe und einer Gasversorgung vor der Rückhaltestufe derart angeordnet, dass das Gas die Mahlvorrichtung entlang ihrer Längsachse durchströmt. Die gewählte Konstruktion dieser Vorrichtung erschwert jedoch die Gewährleistung einer mehr oder weniger einheitlichen Teilchengröße der im herstellten Salzaerosol enthaltenen Salzpartikel. Um zu vermeiden, dass auch unerwünscht große Salzpartikel aus der Vorrichtung ausströmen, müssen hier extra Siebe oder Prallbleche eingesetzt werden.
Die WO 2008/084269 A2, welche die Grundlage für den Oberbegriff des Anspruchs 1 bildet, zeigt eine Vorrichtung zur Herstellung eines hochdispersen Aerosols geringer Feuchtigkeit. Diese Vorrichtung umfasst ein Gebläse sowie einen Luftkanal, der um das Gebläse herum ausgebildet und mit einem elektronischen Lufterhitzer ausgestattet ist. Ferner umfasst die Vorrichtung eine Mahlmühle zum Zerkleinern eines Salzes, wobei die Mahlmühle über einen Extra-Luftkanal mit dem Luftkanal verbunden ist, der um das Gebläse herum ausgebildet ist. Die von der Mahlmühle zerkleinerten Salzpartikel gelangen über diesen Extra-Luftkanal in den um das Gebläse ausgebildeten Luftkanal und werden von dort aus über ein Steigrohr aus der Vorrichtung herausgeblasen.
Aus der WO 2015/006838 A1 ist ferner ein Puderinhalator bekannt. Aus der US 1,894,106 A ist darüber hinaus eine Mühle bekannt, bei der zum Zerkleinern des Mahlguts eine magnetisch angetriebene Kugel eingesetzt wird.

Es ist somit eine Aufgabe der vorliegenden Erfindung, eine Vorrichtung zum Mikronisieren eines anorganischen Salzes und zur Herstellung eines Salzaerosols bereitzustellen, welche die oben genannten Nachteile überwindet. Hierbei ist es insbesondere eine Aufgabe der vorliegenden Erfindung, eine derartige Vorrichtung in Bezug auf die Zuverlässigkeit in der Herstellung von möglichst einheitlichen Teilchengrößen der im Salzaerosol vorhandenen Salzpartikel sowie in Bezug auf die Reinigungsfähigkeit, Hygiene, Geräuschentwicklung, Wartungsintensität und Herstellkosten zu verbessern.

Diese Aufgabe wird durch eine Vorrichtung gemäß Anspruch 1 gelöst, wobei diese Vorrichtung Folgendes umfasst:
- einen Aufnahmebehälter zur Aufnahme des zu mikronisierenden Salzes im Inneren des Aufnahmebehälters;
- eine Mahleinrichtung zur Zerkleinerung des im Aufnahmebehälter befindlichen, zu mikronisierenden Salzes und zur Bildung von mikronisierten Salzpartikeln;
- ein Steigrohr, welches an seinem unteren Ende an den Aufnahmebehälter angeschlossen und mit diesem fluidisch verbunden ist und zum Transport der mikronisierten Salzpartikel dient, wobei ein von dem unteren Ende abgewandtes oberes Ende des Steigrohres eine Auslassöffnung aufweist, durch welche die mikronisierten Salzpartikel aus der Vorrichtung ausströmen können;
- einen Lüfter zur Erzeugung eines Luftstroms; und
- ein Gehäuse mit einem Luftauslass und einem Luftkanal, welcher den Lüfter mit dem Luftauslass verbindet, wobei der Luftkanal durch zumindest eine Wand vom Inneren des Aufnahmebehälters getrennt ist, so dass der vom Lüfter erzeugte Luftstrom das Innere des Aufnahmebehälters nicht durchströmt, und wobei der Luftauslass das obere Ende des Steigrohres im Bereich der Auslassöffnung zumindest teilweise umgibt.

Die erfindungsgemäße Vorrichtung zeichnet sich insbesondere durch die im Vergleich zum Stand der Technik neue Art der Luftführung innerhalb des Gehäuses aus. Der Aufnahmebehälter, in welchen das zu mikronisierende Salz üblicherweise vor Inbetriebnahme der Vorrichtung eingesetzt wird, wird von dem vom Lüfter erzeugten Luftstrom nämlich nicht direkt durchströmt, wie dies bei derartigen Vorrichtungen meist der Fall ist. Stattdessen ist im Gehäuse ein Luftkanal vorgesehen, welcher vom Inneren des Aufnahmebehälters durch zumindest eine Wand getrennt ist. Dieser Luftkanal führt innerhalb des Gehäuses der Vorrichtung vom Lüfter zu einem Luftauslass. Der Luftauslass umgibt zumindest teilweise ein Steigrohr, welches an den Aufnahmebehälter angeschlossen ist und in dem die von der Mahleinrichtung zerkleinerten, mikronisierten Salzpartikel aufsteigen. Genauer gesagt umgibt der Luftauslass das Steigrohr im Bereich der Auslass Öffnung, durch welche die mikronisierten Salzpartikel aus der Vorrichtung ausströmen. Der vom Lüfter erzeugte Luftstrom und die mikronisierten Salzpartikel werden also im Bereich der Auslassöffnung, welche am oberen Ende des Steigrohres angeordnet ist, zusammengeführt.

Durch die oben genannte Anordnung des Luftkanals und die Vermeidung einer direkten Durchströmung des Aufnahmebehälters ergeben sich folgende Vorteile: Zum einen wird durch die Umströmung der Auslassöffnung im Inneren des Steigrohrs eine Sogwirkung erzeugt, welche vorzugsweise lediglich im oberen Teil des Steigrohrs, also nahe der Auslassöffnung, effektiv zum Tragen kommt. Diese Art von Saugströmung, welche im oberen Teil des Steigrohres induziert wird, wirkt sich nur auf die Salzpartikel aus, welche in der Mahleinrichtung bereits genügend stark zerkleinert wurden und aufgrund der ihrer innerhalb der Mahleinrichtung erfahrenen Beschleunigung entgegen der Schwerkraft im Inneren des Aufnahmebehälters und des daran anschließenden Steigrohres bis zu einer gewissen Höhe aufgestiegen sind. Dies wiederum gewährleistet, dass unerwünscht große Salzpartikel von der zuvor genannten Saugströmung gar nicht erfasst werden und somit auch nicht aus der Vorrichtung ausströmen, da diese größeren Salzpartikel aufgrund ihres relativ gesehen höheren Gewichts im Aufnahmebehälter und dem daran anschließenden Steigrohr nicht weit genug aufsteigen. Schlussendlich kann somit also die Einhaltung einer maximalen Teilchengröße der ausströmenden Salzpartikel garantiert werden.

Die erfindungsgemäße Art der Luftführung innerhalb der Vorrichtung hat zudem den Vorteil, dass das in der Vorrichtung befindliche Salz vom Lüfter in gewisser Weise abgeschirmt wird, da der Luftstrom im Sinne eines Bypasses innerhalb der Vorrichtung separat von den Salzpartikeln geleitet wird. Dies wiederum bringt hygienische Vorteile und wirkt sich auch bezüglich der Wartungsintensität durch eine geringere Korrosionsanfälligkeit positiv aus.

Ein weiterer Vorteil der erfindungsgemäßen Vorrichtung ist in deren vergleichsweise kostengünstiger Herstellung sowie der vergleichsweise einfachen Reinigungsmöglichkeit der Vorrichtung zu sehen, da beispielsweise im Vergleich zu der aus der EP 2 457 559 A1 bekannten Vorrichtung keine extra Siebe oder Prallbleche im Steigrohr vorgesehen werden müssen.

Gemäß einer Ausgestaltung der vorliegenden Erfindung ist der Luftauslass des Luftkanals konzentrisch zu der Auslassöffnung des Steigrohres angeordnet, wobei der Luftauslass die Auslassöffnung vollständig umgibt.

Diese Ausgestaltung hat den Vorteil, dass die oben beschriebene Saugströmung, welche im oberen Bereich des Steigrohres induziert wird, möglichst über den gesamten Durchmesser des Steigrohres ihre Wirkung entfaltet. Dies führt zu einer relativ guten Verteilung der aus der Vorrichtung ausströmenden, mikronisierten Salzpartikel. Ferner lässt sich auch die Intensität, mit der die mikronisierten Salzpartikel aus der Vorrichtung ausströmen, durch das oben erwähnte Prinzip relativ gut regeln.

Gemäß einer weiteren Ausgestaltung der vorliegenden Erfindung weist die Mahleinrichtung (i) einen Motor, (ii) einen vom Motor rotatorisch angetriebenen Magneten sowie (iii) eine Kugel aus magnetisierbarem Material auf, wobei die Kugel innerhalb des Aufnahmebehälters angeordnet ist und der Motor sowie der davon angetriebene Magnet außerhalb des Aufnahmebehälters angeordnet ist, und wobei die Kugel durch die Rotation des Magneten relativ zu dem Aufnahmebehälter bewegt wird. Dabei ist es insbesondere bevorzugt, dass die Mahleinrichtung genau eine Kugel aus magnetisierbarem Material aufweist.

Diese Ausgestaltung hat den Vorteil, dass hierdurch ein berührungsloser Antrieb der Mahleinrichtung gewährleistet ist, wobei unter "berührungslos" hierbei verstanden wird, dass es keinen direkten, mechanischen Kontakt zwischen der im Aufnahmebehälter befindlichen Kugel und dem üblicherweise unter dem Aufnahmebehälter angeordneten Antrieb mit Motor und dem damit verbundenen Magneten gibt. Dies ist wiederum aus hygienischen Gründen von Vorteil, da es nicht zu einer Verunreinigung des im Aufnahmebehälter befindlichen Salzes durch Schmierfette oder Öle kommen kann, welche im Bereich des Antriebs vorzufinden sind. Im Übrigen kann dadurch auch die Korrosionsgefahr an den Antriebsteilen der Mahleinrichtung drastisch reduziert werden. Als weiterhin vorteilhaft hat sich herausgestellt, dass diese Art von Mahleinrichtung mit magnetisch angetriebener Kugel im Vergleich zu sonstigen, aus dem Stand der Technik bekannten Mahlvorrichtungen relativ leise ist.

Bei der genannten Verwendung einer Mahleinrichtung mit magnetisch angetriebener Kugel sorgen mehrere Effekte für die Zerkleinerung des zu mikronisierenden Salzes bzw. zur Bildung der mikronisierten Salzpartikel: Zum einen werden die Salzkörner durch die im Aufnahmebehälter rotierende Kugel in Bewegung versetzt, wodurch diese gegeneinander stoßen und auch auf die Innenwand des Aufnahmebehälters treffen. Die Salzkörner zerstoßen und zerreiben sich dadurch gegenseitig. In geringerem Maße werden die Salzkörner auch zwischen der Kugel und der Innenwand des Aufnahmebehälters zermahlen.

Gemäß einer bevorzugten Ausgestaltung hat der Aufnahmebehälter eine geschlossene, runde Bodenfläche.

Dies garantiert, dass sich die Kugel mit relativ hohen Geschwindigkeiten auf einer Kreisbahn bewegen kann. Aufgrund der während des rotatorischen Antriebs der Kugel auf die Kugel einwirkende Zentrifugalkraft bewegt sich diese üblicherweise entlang des äußeren Randes der Bodenfläche gestützt durch die Innenwände des Aufnahmebehälters.

Gemäß einer weiteren Ausgestaltung ist es vorgesehen, dass der Durchmesser der Bodenfläche zumindest dem 5-Fachen, vorzugsweise zumindest dem 10-Fachen des Durchmessers der Kugel entspricht.

Die Kugel ist also vorzugsweise sehr klein im Vergleich zum Durchmesser des Aufnahmebehälters gewählt. Der Aufnahmebehälter selbst ist vorzugsweise zylindrisch geformt. Eine konische Form des Aufnahmebehälters wäre jedoch ebenso denkbar.

Gemäß einer weiteren Ausgestaltung weist der Motor eine Motorwelle auf, an welcher der Magnet exzentrisch angeordnet ist, wobei die Mahleinrichtung ferner ein Ausgleichsgewicht aufweist, welches in etwa dem Gewicht des Magneten, also zwischen 90 % und 110 % des Gewichts des Magneten, entspricht und auf einer dem Magneten gegenüberliegenden Seite exzentrisch an der Motorwelle angeordnet ist.

Diese Ausgestaltung hat den Vorteil, dass Unwuchten am Antrieb vermieden werden. Somit lassen sich höhere Drehzahlen gewährleisten. Ebenso kann hierdurch auch die durch den Antrieb verursachte Geräuschentwicklung minimiert werden. Es versteht sich ohnehin, dass hierdurch auch die mechanische Gesamtstabilität des Antriebs verbessert wird.

Die erfindungsgemäße Vorrichtung weist ferner vorzugsweise eine Steuereinheit zur Steuerung des Motors auf, wobei die Steuereinheit dazu eingerichtet ist, den Motor mit einer ersten Nenndrehzahl zu betreiben, wobei die erste Nenndrehzahl zwischen 1.500 und 2.500 Umdrehungen pro Minute gewählt ist.

Es sei darauf hingewiesen, dass der Begriff "erste Nenndrehzahl" vorliegend lediglich dazu verwendet wird, um diese von einer nachfolgend noch erläuterten "zweiten Nenndrehzahl" zu unterscheiden. Diese Terminologie soll jedoch keinerlei Reihenfolge, Priorität oder andere Art von Bedeutung implizieren.

Als besonders bevorzugt hat sich eine Nenndrehzahl von 1.800 bis 2.800 Umdrehungen pro Minute herausgestellt. Es hat sich gezeigt, dass sich in diesem Drehzahlbereich ein optimaler Zerkleinerungseffekt der im Aufnahmebehälter befindlichen Salzkristalle ergibt.

Gemäß einer weiteren Ausgestaltung ist die Steuereinheit dazu eingerichtet, die Nenndrehzahl des Motors in regelmäßigen Abständen vorübergehend auf eine zweite Nenndrehzahl zu verändern und jeweils anschließend wieder zu der ersten Nenndrehzahl zurückzukehren, wobei die zweite Nenndrehzahl größer ist als die erste Nenndrehzahl.

Der vom Motor angetriebene Magnet wird also in regelmäßigen zeitlichen Abständen vorübergehend stärker beschleunigt, vorzugsweise über die Maximaldrehzahl der oben genannten Bereiche hinaus. Dies hat zur Folge, dass die auf die Kugel einwirkende Magnetkraft nicht mehr ausreicht, um die Kugel zur gemeinsamen Rotation mit dem vom Motor angetriebenen Magneten zu zwingen. Die Kugel kann somit dem rotierenden Magneten nicht mehr exakt folgen und wird von ihrer Kreisbahn abgebracht. Hierdurch kann es zu einer Art Taumelbewegung der Kugel innerhalb des Aufnahmebehälters kommen. Dieses Vorgehen hat insbesondere den Vorteil, dass hierdurch Ansammlungen von Salz in der Mitte des Aufnahmebehälters von der Kugel mitgerissen werden und dem Zerkleinerungsprozess zugeführt werden. Durch die anschließende Rückkehr auf die erste Nenndrehzahl wird die Kugel wieder auf ihre Kreisbahn innerhalb des Aufnahmebehälters zurückgebracht.

Vorzugsweise weist die Vorrichtung eine Eingabeeinrichtung zur Definition der ersten Nenndrehzahl durch den Nutzer auf. Dies erlaubt dem Nutzer eine Regelung der Intensität, mit welcher das erzeugte Salzaerosol aus der Vorrichtung ausströmt. So kann die Menge des ausströmenden Salzaerosols beispielsweise an die Größe des Raums angepasst werden, in welchem die Vorrichtung aufgestellt ist. Denkbar ist die Einstellung der ersten Nenndrehzahl über einen oder mehrere Wählschalter.

Alternativ oder zusätzlich zu der zuletzt genannten Ausgestaltung kann die Vorrichtung einen Temperatursensor zur Erzeugung eines Temperatursignals und ein Hygrometer zur Erzeugung eines Feuchtigkeitssignals aufweisen, wobei die Steuereinheit dazu eingerichtet ist, die erste Nenndrehzahl in Abhängigkeit des Temperatursignals und des Feuchtigkeitssignals zu regeln. Bevorzugt ist die Steuereinheit dazu eingerichtet, den Motor bei Überschreitung eines vordefinierten Temperatur- und/oder Feuchtigkeits-Schwellwerts abzuschalten. Dies dient insbesondere der Vermeidung von Beschädigungen sowie der Vermeidung von Fehlfunktionen.

Diese Ausgestaltung hat den Vorteil, dass eine Regelung der Intensität des aus der Vorrichtung ausströmenden Salzaerosols automatisch erfolgt. Dies ist insbesondere deshalb vorteilhaft, da das Umgebungsklima entscheidend ist für die Eigenschaften des Salzaerosols. Ein zu feuchtes Raumklima verhindert die Funktionsweise der erfindungsgemäßen Vorrichtung. Ein zu heißes Umgebungsklima kann zu Beeinträchtigungen verschiedener elektrischer und mechanischer Teile der Vorrichtung führen. Zur Vermeidung derartiger Fehlfunktionen oder Beschädigungen kann es daher vorgesehen sein, dass die Steuereinheit dazu eingerichtet ist, die Vorrichtung bei Erreichen eines Temperatur- und/oder Feuchtigkeitsschwellwerts abzuschalten.

Das Steigrohr ist gemäß einer weiteren Ausgestaltung der vorliegenden Erfindung vorzugsweise gekrümmt.

Die Krümmung des Steigrohres bietet insbesondere die Möglichkeit einer platzsparenden Anordnung, ohne die effektive Länge des Steigrohres verkürzen zu müssen. Bei einer Krümmung des Steigrohres um 90° kann der an den Aufnahmebehälter angeschlossenen unteren Teil des Steigrohres vertikal ausgerichtet sein, wobei die aus der Vorrichtung austretenden, mikronisierten Salzpartikel horizontal aus der am oberen Ende des Steigrohres angeordneten Auslassöffnung austreten.

Gemäß einer weiteren Ausgestaltung ist der Aufnahmebehälter lösbar am Gehäuse angeordnet.

Zur Befüllung des Aufnahmebehälters mit Salz kann dieser somit vom Gehäuse getrennt werden. Dies vereinfacht auch die Reinigungsmöglichkeit des Aufnahmebehälters.

Gemäß einer weiteren Ausgestaltung der vorliegenden Erfindung ist es ferner vorgesehen, dass die Vorrichtung eine Beleuchtung zur Beleuchtung des Inneren des Aufnahmebehälters und/oder zur Beleuchtung der Auslassöffnung aufweist.

Die Beleuchtung des Inneren des Aufnahmebehälters hat den Vorteil, dass sich der Füllstand des im Aufnahmebehälter befindlichen Salzes durch den Benutzer leicht einsehen lässt. Durch eine Beleuchtung der Auslassöffnung können die aus der Vorrichtung austretenden, mikronisierten Salzpartikel besser wahrgenommen werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in den nachfolgenden Zeichnungen dargestellt und wird in der nachfolgenden Beschreibung näher erläutert. Es zeigen:
- Fig. 1: eine perspektivische Ansicht eines Ausführungsbeispiels der erfindungsgemäßen Vorrichtung;
- Fig. 2: eine weitere perspektivische Ansicht des in Fig. 1 gezeigten Ausführungsbeispiels der erfindungsgemäßen Vorrichtung, wobei im Vergleich zu Fig. 1 ein zu der Vorrichtung gehöriger Aufnahmebehälter vom Gehäuse der Vorrichtung gelöst ist;
- Fig. 3: eine Draufsicht von vorne auf das in Fig. 1 gezeigte Ausführungsbeispiel der erfindungsgemäßen Vorrichtung;
- Fig. 4: eine Querschnittansicht des in Fig. 1 gezeigten Ausführungsbeispiels der erfindungsgemäßen Vorrichtung;
- Fig. 5: eine Längsschnittansicht des in Fig. 1 gezeigten Ausführungsbeispiels der erfindungsgemäßen Vorrichtung; und
- Fig. 6: eine perspektivische Innenansicht des in Fig. 1 gezeigten Ausführungsbeispiels der erfindungsgemäßen Vorrichtung, wobei das Gehäuse ausgeblendet bzw. nur schematisch dargestellt ist.

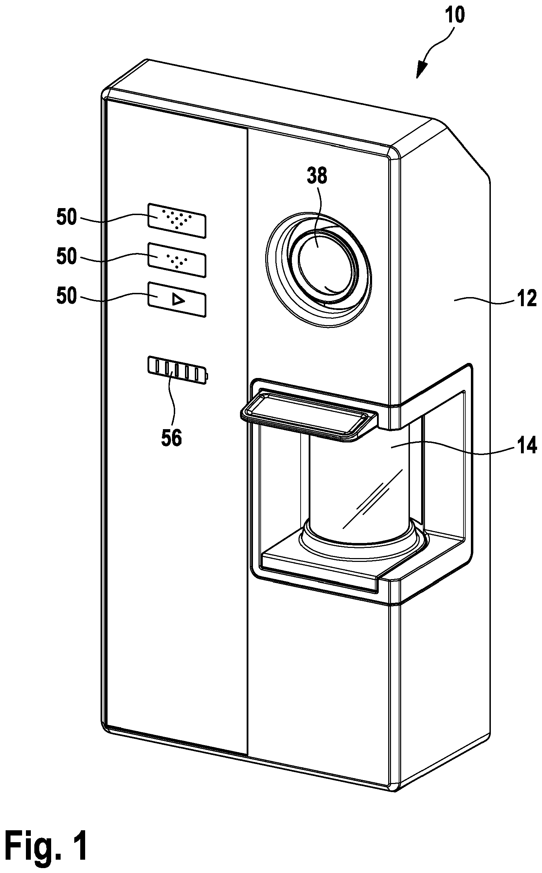

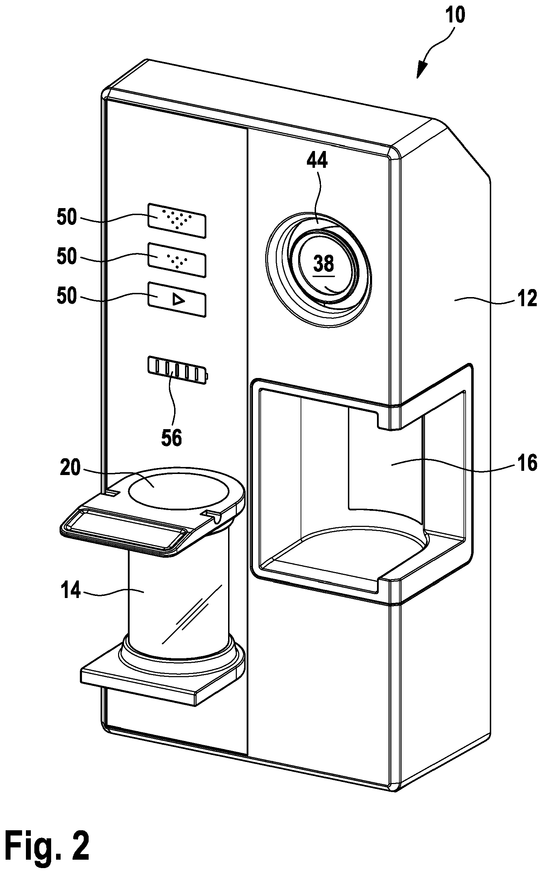

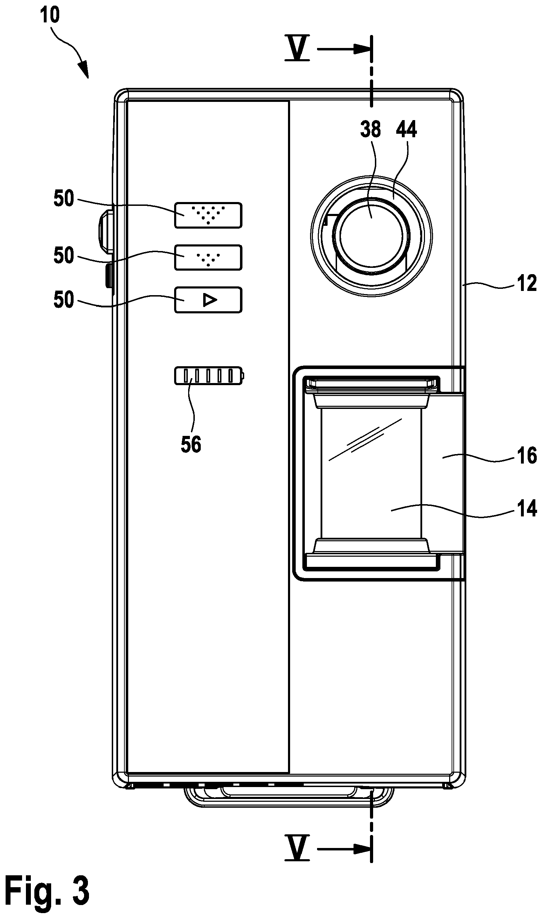

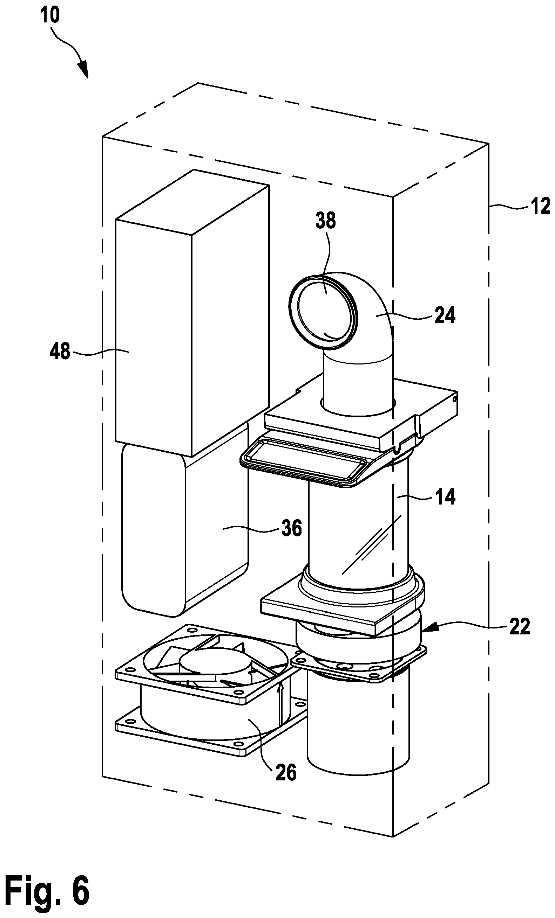

Die Fig. 1-6 zeigen unterschiedliche Ansichten eines Ausführungsbeispiels der erfindungsgemäßen Vorrichtung. Die Vorrichtung ist darin in ihrer Gesamtheit mit der Bezugsziffer 10 bezeichnet.

Die Vorrichtung 10 weist ein Gehäuse 12 sowie einen Aufnahmebehälter 14 auf, welcher vorzugsweise lösbar an dem Gehäuse 12 befestigt werden kann. Hierzu ist auf der Vorderseite des Gehäuses 12 eine Vertiefung bzw. Ausnehmung 16 vorgesehen, in welche der Aufnahmebehälter 14 vorzugsweise formschlüssig eingesetzt werden kann.

Der Aufnahmebehälter 14 dient zur Aufnahme von Salzkörnern 46, welche mithilfe der Vorrichtung 10 zur Herstellung eines Salzaerosols mikronisiert werden sollen. Der Aufnahmebehälter 14 hat vorzugsweise eine zylindrische Form mit einer geschlossenen, kreisrunden Bodenfläche 18. Nach oben hin ist der Aufnahmebehälter 14 vorzugsweise offen. Durch eine Öffnung 20 können die im Aufnahmebehälter 14 zerkleinerten Salzpartikel 54 während des Betriebs der Vorrichtung 10 somit nach oben hin aus dem Aufnahmebehälter 14 austreten.

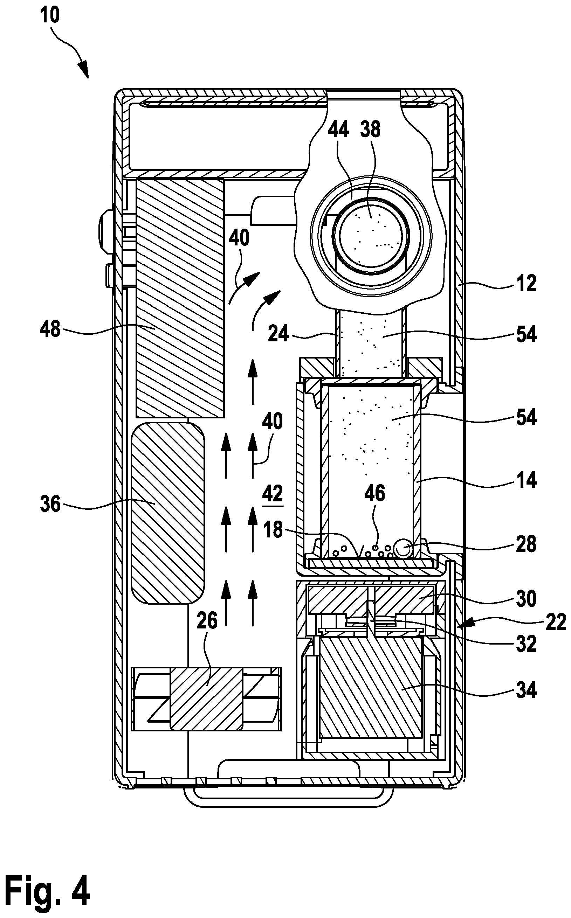

Die erfindungsgemäße Vorrichtung 10 weist ferner eine Mahleinrichtung 22, ein Steigrohr 24 und einen Lüfter 26 auf, welche vorzugsweise allesamt innerhalb des Gehäuses 12 angeordnet sind (siehe Fig. 4-6).

Die Mahleinrichtung 22 dient zur Verkleinerung des im Aufnahmebehälter 14 befindlichen, zu mikronisierenden Salzes 46, also zur Bildung von mikronisierten Salzpartikeln 54. Die Mahleinrichtung 22 weist in dem vorliegend dargestellten Ausführungsbeispiel eine magnetisch angetriebene Kugel 28 auf, welche lösbar in den Aufnahmebehälter 14 eingesetzt ist. Die Kugel 28 wird mithilfe eines rotatorisch angetriebenen Magneten 30 während des Betriebs der Vorrichtung 10 innerhalb des Aufnahmebehälters 14 auf einer Kreisbahn bewegt. Der Magnet 30 ist hierzu exzentrisch auf einer Motorwelle 32 eines elektrischen Antriebsmotors 34 befestigt. Die Spannungsversorgung des Elektromotors 34 erfolgt vorzugsweise über einen ebenfalls im Gehäuse 12 integrierten Energiespeicher 36, welcher zum Beispiel einen Akkumulator umfasst.

Das Steigrohr 24 ist fluidisch mit dem Inneren des Aufnahmebehälters 14 verbunden. Es dient dem Transport der durch die Mahleinrichtung 22 im Inneren des Aufnahmebehälters 14 mikronisierten Salzpartikel nach außen aus der Vorrichtung 10 hinaus. In Strömungsrichtung der mikronisierten Salzpartikel betrachtet ist das Steigrohr 24 somit stromabwärts relativ zu dem Aufnahmebehälter 14 angeordnet. Der untere Teil des Steigrohrs 24, welcher an den Aufnahmebehälter 14 angeschlossen ist, ist vorzugsweise koaxial zu dem Aufnahmebehälter 14 angeordnet. Der obere Teil des Steigrohrs 24 ist vorzugsweise gekrümmt. Wenngleich grundsätzlich auch ein ungekrümmtes Steigrohr in Betracht käme, so hat ein gekrümmtes Steigrohr im Vergleich zu einem ungekrümmten Steigrohr den Vorteil einer geringeren Bauhöhe bei gleicher effektiver Länge.

An dem vom Aufnahmebehälter 14 abgewandten Ende weist das Steigrohr 24 eine Auslassöffnung 38 auf, durch welche die mikronisierten Salzpartikel 54 aus der Vorrichtung 10 nach außen ausströmen können.

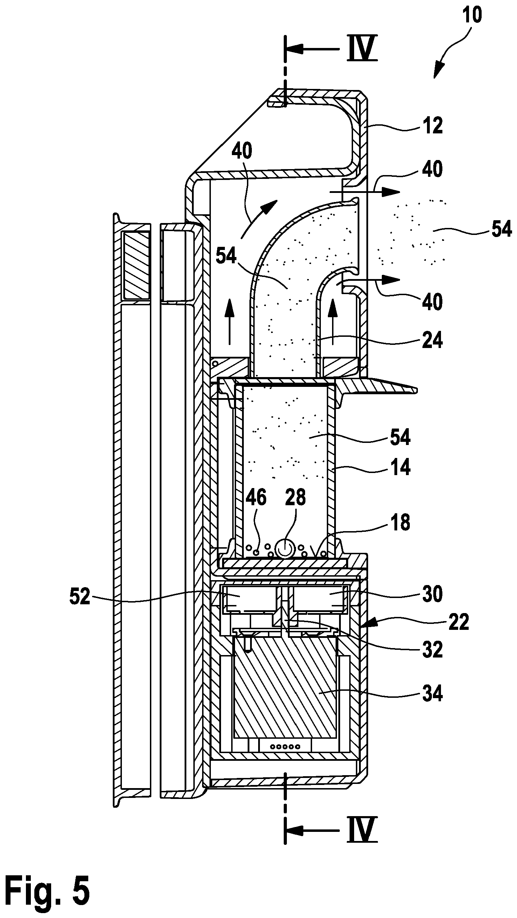

Der Lüfter 26, welcher vorzugsweise als Ventilator ausgestaltet ist, erzeugt im Inneren des Gehäuses 12 einen Luftstrom, welcher schematisch mit Pfeilen 40 angedeutet ist (siehe Fig. 4 und 5). Der Lüfter 26 ist vorzugsweise dazu eingerichtet, einen Volumenstrom im Bereich von 10-20 m³/h zu erzeugen. Ein wesentliches Merkmal dieses Luftstroms 40 besteht darin, dass dieser nicht, wie meist für derartige Mikronisatoren üblich, durch den Aufnahmebehälter 14 hindurchströmt, sondern im Inneren des Gehäuses 12 durch einen hier nicht näher im Detail dargestellten Luftkanal 42 am Aufnahmebehälter 14 vorbeigeleitet wird. Der Luftkanal 42 mündet an der dem Lüfter 26 entgegengesetzten Ende in einen Luftauslass 44. Dieser Luftauslass 44 ist vorzugsweise konzentrisch zu der Auslassöffnung 38 des Steigrohrs 24 angeordnet. Der Luftauslass 44 umgibt die Auslassöffnung 38 vorzugsweise vollständig. Sowohl die Auslassöffnung 38 als auch der Luftauslass 44 haben vorzugsweise eine runde Außenkontur. Durch deren konzentrische Anordnung ergibt sich somit ein in etwa kreisringförmiger Querschnitt des Luftauslasses 44.

Die Funktionsweise der Vorrichtung 10 lässt sich am besten anhand der Fig. 4 und 5 nachvollziehen.

Zunächst wird das vor Betriebsbeginn in den Aufnahmebehälter 14 eingegebene Salz 46 mithilfe der Mahleinrichtung 22 zerkleinert bzw. mikronisiert. Hierzu wird mittels des Elektromotors 34 der Magnet 30 rotiert. Der Motor 34 wird dabei von einer Steuereinheit 48 gesteuert, welche die Nenndrehzahl des Motors vorzugsweise auf 1.500 bis 2.500 Umdrehungen pro Minute regelt. Über Wählschalter 50, welche auf der Außenseite des Gehäuses 12 angebracht sind und vorliegend allgemein als Eingabeeinrichtung bezeichnet werden, ist die Drehzahl des Motors 34 seitens des Nutzers stufenweise einstellbar. Hierdurch lässt sich die Intensität des erzeugten Salzaerosols seitens des Nutzers aktuell einstellen. Grundsätzlich ist auch eine stufenlose Einstellung der Drehzahl, beispielsweise durch einen Drehschalter, denkbar.

Durch die Rotation des Magneten 30 wird die im Aufnahmebehälter 14 befindliche Kugel 28 mitgerissen, so dass diese in etwa synchron zu dem Magneten 30 innerhalb des Aufnahmebehälters 14 rotiert. Zum Ausgleich der durch die Bewegung des Magneten 30 entstehenden Fliehkräfte ist auf der Motorwelle 32 auf der dem Magneten 30 gegenüberliegenden Seite vorzugsweise ein Ausgleichsgewicht 52 angebracht. Anstelle dieses Ausgleichsgewichts 52 kann an dieser Position auch ein zweiter Magnet angeordnet sein.

Der durch die Bewegung der Kugel 28 verursachte Zerkleinerungsprozess der Salzkristalle 46 basiert auf mehreren Effekten: Zum einen werden die Salzkristalle 46 durch die Rotation der Kugel 28 in Bewegung versetzt und innerhalb des Aufnahmebehälters 14 umhergeschleudert. Manche Salzkristalle prallen hierdurch gegenseitig aufeinander. Andere wiederum prallen dadurch gegen die Innenwand des Aufnahmebehälters 14. Wieder andere Salzkristalle werden zwischen der Kugel 28 und der Innenwand des Aufnahmebehälters 14 zermahlen. Es hat sich gezeigt, dass es durch die Gemeinsamkeit dieser Effekte zu einem sehr effizienten Verkleinerungsprozess der Salzkristalle 46 kommt.

Die zerkleinerten bzw. mikronisierten Salzkristalle, welche in Fig. 4 und 5 schematisch angedeutet sind und mit der Bezugsziffer 54 versehen sind, werden durch die Bewegung der Kugel 28 innerhalb des Aufnahmebehälters 14 nach oben geschleudert. Sofern diese klein genug sind, können sie entgegen der wirkenden Gewichtskraft im Aufnahmebehälter 14 und innerhalb des daran anschließenden Steigrohrs 24 aufsteigen. Der vom Lüfter 26 erzeugte Luftstrom 40 erzeugt aufgrund der oben beschriebenen Umströmung des Steigrohrs 24 im Bereich der Auslassöffnung 38 eine Art Saugströmung. Diese Saugströmung wird im Inneren des Steigrohrs 24 insbesondere im Bereich der Auslassöffnung 38 induziert. Somit ist gewährleistet, dass nur die mikronisierten Salzpartikel 54, welche innerhalb des Aufnahmebehälters 14 und innerhalb des Steigrohrs 24 entgegen der wirkenden Gewichtskraft aufgestiegen sind, von der Saugströmung erfasst werden und aus der Auslassöffnung 38 und damit aus der Vorrichtung 10 ausströmen. Größere Salzpartikel hingegen, welche aufgrund ihres Gewichts am Boden des Aufnahmebehälters 14 verbleiben oder zumindest nicht bis in den oberen Bereich des Steigrohrs 24 aufsteigen, werden durch den vom Luftstrom 40 im Steigrohr 24 erzeugten Sog nicht erfasst und verbleiben somit in der Vorrichtung 10.

Um zu vermeiden, dass sich auf der Bodenmitte des Aufnahmebehälters 14 größere Salzkristalle dauerhaft ablagern, welche von der rotierenden Kugel nicht erfasst werden, ist es vorzugsweise vorgesehen, dass die Steuereinheit 48 in regelmäßigen zeitlichen Abständen die Nenndrehzahl des Motors 34 vorübergehend über einen definierten Schwellwert erhöht. Sofern nämlich eine gewisse Drehzahl des Motors 34 überschritten wird, ist die zwischen der Kugel 28 und dem Magnet 30 wirkende Kraft nämlich nicht mehr ausreichend dafür, dass die Kugel 28 der Rotation des Magneten 30 folgen kann. Die Kugel 28 beginnt dann auf der Bodenfläche 18 des Aufnahmebehälters 14 zu "tänzeln". Dabei bewegt sie sich über Bereiche der Bodenfläche 18, insbesondere auch die Mitte der Bodenfläche 18, welche sie während ihrer üblichen Rotation entlang des Außenrands der Bodenfläche 18 sonst nicht erreicht. Die an diesen Stellen befindlichen Salzkristalle werden dann also ebenfalls erfasst. Die oben genannte vorübergehende Drehzahlerhöhung erfolgt vorzugsweise nur wenige Sekunden.

Weiterhin können folgende Merkmale vorgesehen sei: Auf der Außenseite des Gehäuses 12 kann eine Anzeige 56 zur Anzeige des Füllstands des Akkumulators 36 vorgesehen sein. Zudem kann die Vorrichtung 10 optional eine der Einfachheit halber in den Zeichnungen nicht gezeigte Beleuchtung aufweisen, welche zur Beleuchtung der Auslassöffnung 38 und/oder des Inneren des Aufnahmebehälters 14 dient. Des Weiteren weist die Vorrichtung 10 optional einen Temperatursensor sowie einen Hygrometer auf, wobei die Steuereinheit 48 in diesem Fall dazu eingerichtet ist, die Drehzahl des Motors 34 in Abhängigkeit des vom Temperatursensor erzeugten Temperatursignals und/oder des vom Hygrometer erzeugten Feuchtigkeitssignals zu regeln. Bevorzugt ist die Steuereinheit 48 dazu eingerichtet, den Motor 34 bei Überschreitung eines vordefinierten Temperatur- und/oder Feuchtigkeits-Schwellwerts abzuschalten. Dies dient insbesondere der Vermeidung von Beschädigungen sowie der Vermeidung von Fehlfunktionen.

## Patentansprüche

1. Vorrichtung (10) zum Mikronisieren eines anorganischen Salzes, mit:
- einem Aufnahmebehälter (14) zur Aufnahme des zu mikronisierenden Salzes (46) im Inneren des Aufnahmebehälters (14);
- einer Mahleinrichtung (22) zur Zerkleinerung des im Aufnahmebehälter (14) befindlichen, zu mikronisierenden Salzes (46) und zur Bildung von mikronisierten Salzpartikeln (54);
- einem Steigrohr (24), welches an seinem unteren Ende an den Aufnahmebehälter (14) angeschlossen und mit diesem fluidisch verbunden ist und zum Transport der mikronisierten Salzpartikel (54) dient, wobei ein von dem unteren Ende abgewandtes oberes des Steigrohres (24) eine Auslassöffnung (38) aufweist, durch welche die mikronisierten Salzpartikel (54) aus der Vorrichtung (10) ausströmen können;
- einem Lüfter (26) zur Erzeugung eines Luftstroms (40); und
- einem Gehäuse (12) mit einem Luftauslass (44) und einem Luftkanal (42), welcher den Lüfter (26) mit dem Luftauslass (44) verbindet, wobei der Luftkanal (42) durch zumindest eine Wand vom Inneren des Aufnahmebehälters (14) getrennt ist, so dass der vom Lüfter (26) erzeugte Luftstrom (40) das Innere des Aufnahmebehälters (14) nicht durchströmt,
**dadurch gekennzeichnet, dass** der Luftauslass (44) das obere Ende des Steigrohres (24) im Bereich der Auslassöffnung (38) zumindest teilweise umgibt.

2. Vorrichtung nach Anspruch 1, wobei der Luftauslass (44) konzentrisch zu der Auslassöffnung (38) angeordnet ist und diese vollständig umgibt.

3. Vorrichtung nach Anspruch 1 oder 2, wobei die Mahleinrichtung (22) (i) einen Motor (34), (ii) einen von dem Motor (34) rotatorisch angetriebenen Magneten (30) sowie (iii) eine Kugel (28) aus magnetisierbarem Material aufweist, wobei die Kugel (28) in dem Aufnahmebehälter (14) angeordnet ist und der Motor (34) sowie der Magnet (30) außerhalb des Aufnahmebehälters (14) angeordnet sind.

4. Vorrichtung nach Anspruch 3, wobei die Mahleinrichtung (22) genau eine Kugel (28) aus magnetisierbarem Material aufweist.

5. Vorrichtung nach Anspruch 3 oder 4, wobei der Aufnahmebehälter (14) eine geschlossene, runde Bodenfläche (18) hat.

6. Vorrichtung nach Anspruch 5, wobei der Durchmesser der Bodenfläche (18) zumindest dem 5-Fachen, vorzugsweise zumindest dem 10-Fachen des Durchmessers der Kugel (28) entspricht.

7. Vorrichtung nach einem der Ansprüche 3 bis 6, wobei der Motor (34) eine Motorwelle (32) aufweist, an welcher der Magnet (30) exzentrisch angeordnet ist.

8. Vorrichtung nach Anspruch 7, wobei die Mahleinrichtung (22) ein Ausgleichsgewicht (52) aufweist, welches in etwa dem Gewicht des Magneten (30), also zwischen 90% und 110% des Gewichts des Magneten (30), entspricht und auf einer dem Magneten (30) gegenüberliegenden Seite, exzentrisch an der Motorwelle (32) angeordnet ist.

9. Vorrichtung nach einem der Ansprüche 3 bis 8, wobei die Vorrichtung (10) eine Steuereinheit (48) zur Steuerung des Motors (34) aufweist, wobei die Steuereinheit (48) dazu eingerichtet ist, den Motor (34) mit einer ersten Nenndrehzahl zu betrieben, wobei die erste Nenndrehzahl zwischen 1.500 und 2.500 Umdrehungen pro Minute ist.

10. Vorrichtung nach Anspruch 9, wobei die Steuereinheit (48) dazu eingerichtet ist, die Nenndrehzahl in regelmäßigen zeitlichen Abständen vorübergehend auf eine zweite Nenndrehzahl zu verändern und jeweils anschließend wieder zu der ersten Nenndrehzahl zurückzukehren, wobei die zweite Nenndrehzahl größer ist als die erste Nenndrehzahl.

11. Vorrichtung nach Anspruch 9 oder 10, wobei die Vorrichtung (10) eine Eingabeeinrichtung (50) zur Definition der ersten Nenndrehzahl durch den Nutzer aufweist.

12. Vorrichtung nach Anspruch 9 oder 10, wobei die Vorrichtung (10) einen Temperatursensor zur Erzeugung eines Temperatursignals und ein Hygrometer zur Erzeugung eines Feuchtigkeitssignals aufweist, wobei die Steuereinheit (48) dazu eingerichtet ist, die erste Nenndrehzahl in Anhängigkeit des Temperatursignals und des Feuchtigkeitssignals zu regeln.

13. Vorrichtung nach einem der Ansprüche 1 bis 12, wobei das Steigrohr (24) gekrümmt ist.

14. Vorrichtung nach einem der Ansprüche 1 bis 13, wobei der Aufnahmebehälter (14) lösbar am Gehäuse (12) angeordnet ist.

15. Vorrichtung nach einem der Ansprüche 1 bis 14, wobei die Vorrichtung (10) eine Beleuchtung zur Beleuchtung des Inneren des Aufnahmebehälters (14) und/oder zur Beleuchtung der Auslassöffnung (38) aufweist.

## Claims

1. An apparatus (10) for micronizing an inorganic salt, comprising:
- a receiving vessel (14) for receiving the salt (46) to be micronized in an interior of the receiving vessel (14);
- a grinding unit (22) for comminuting the salt (46) to be micronized located in the receiving vessel (14) and for forming micronized salt particles (54);
- an ascending pipe (24), which is at its lower end connected to the receiving vessel (14) and fluidically connected thereto and serves to transport the micronized salt particles (54), wherein an upper end of the ascending pipe (24) which is remote from the lower end comprises an outlet orifice (38) through which the micronized salt particles (54) can flow out of the apparatus (10);
- a fan (26) for generating an air stream (40); and
- a housing (12) with an air outlet (44) and an air duct (42) connecting the fan (26) to the air outlet (44), wherein the air duct (42) is separated by at least one wall from the interior of the receiving vessel (14), such that the air stream (40) generated by the fan (26) does not flow through the interior of the receiving vessel (14),
**characterized in that** the air outlet (44) at least partly surrounds the upper end of the ascending pipe (24) in a region of the outlet orifice (38).

2. The apparatus as claimed in claim 1, wherein the air outlet (44) is arranged concentrically with the outlet orifice (38) and completely surrounds it.

3. The apparatus as claimed in claim 1 or 2, wherein the grinding unit (22) comprises (i) a motor (34), (ii) a magnet (30) driven rotationally by the motor (34) and (iii) a ball (28) of magnetizable material, wherein the ball (28) is arranged in the receiving vessel (14) and the motor (34) and the magnet (30) are arranged outside the receiving vessel (14).

4. The apparatus as claimed in claim 3, wherein the grinding unit (22) comprises precisely one ball (28) of magnetizable material.

5. The apparatus as claimed in claim 3 or 4, wherein the receiving vessel (14) has a closed, round bottom face (18).

6. The apparatus as claimed in claim 5, wherein the diameter of the bottom face (18) corresponds to at least 5 times, preferably at least 10 times the diameter of the ball (28).

7. The apparatus as claimed in one of claims 3 to 6, wherein the motor (34) has a motor shaft (32) on which the magnet (30) is eccentrically arranged.

8. The apparatus as claimed in claim 7, wherein the grinding unit (22) has a counterweight (52) which corresponds approximately to the weight of the magnet (30), i.e. to between 90 % and 110 % of the weight of the magnet (30), and is arranged eccentrically on the motor shaft (32) on an opposite side from the magnet (30).

9. The apparatus as claimed in one of claims 3 to 8, wherein the apparatus (10) comprises a control unit (48) for controlling the motor (34), wherein the control unit (48) is designed to operate the motor (34) at a first nominal speed, wherein the first nominal speed is selected to be between 1,500 and 2,500 revolutions per minute.

10. The apparatus as claimed in claim 9, wherein the control unit (48) is designed to change the nominal speed at regular intervals temporarily to a second nominal speed and then in each case to return to the first nominal speed, wherein the second nominal speed is greater than the first nominal speed.

11. The apparatus as claimed in claim 9 or 10, wherein the apparatus (10) comprises an input device (50) for the user to define the first nominal speed.

12. The apparatus as claimed in claim 9 or 10, wherein the apparatus (10) comprises a temperature sensor for generating a temperature signal and a hygrometer for generating a humidity signal, wherein the control unit (48) is designed to regulate the first nominal speed as a function of the temperature signal and of the humidity signal.

13. The apparatus as claimed in one of claims 1 to 12, wherein the ascending pipe (24) is curved.

14. The apparatus as claimed in one of claims 1 to 13, wherein the receiving vessel (14) is arranged detachably on the housing (12).

15. The apparatus as claimed in one of claims 1 to 14, wherein the apparatus (10) comprises lighting for illuminating the interior of the receiving vessel (14) and/or for illuminating the outlet orifice (38).

## Revendications

1. Dispositif (10) pour microniser un sel inorganique, avec:
- un récipient de réception (14) destiné à recevoir le sel à microniser (46) à l'intérieur du récipient de réception (14);
- un dispositif de broyage (22) pour le concassage du sel à microniser (46) se trouvant dans le récipient de réception (14) et pour la formation de particules de sel micronisées (54);
- une colonne montante (24), qui est connectée par son extrémité inférieure au récipient de réception (14) et qui est fluidiquement raccordée à celui-ci et qui sert pour le transport des particules de sel micronisées (54), dans lequel une supérieure de la colonne montante (24) opposée à l'extrémité inférieure présente une ouverture de sortie (38), par laquelle les particules de sel micronisées (54) peuvent s'écouler hors du dispositif (10);
- un ventilateur (26) pour produire un courant d'air (40); et
- un boîtier (12) avec une sortie d'air (44) et un canal d'air (42), qui relie le ventilateur (26) à la sortie d'air (44), dans lequel le canal d'air (42) est séparé par au moins une paroi de l'intérieur du récipient de réception (14), de telle manière que le courant d'air (40) produit par le ventilateur (26) ne traverse pas l'intérieur du récipient de réception (14);
**caractérisé en ce que** la sortie d'air (44) entoure au moins en partie l'extrémité supérieure de la colonne montante (24) dans la région de l'ouverture de sortie (38).

2. Dispositif selon la revendication 1, dans lequel la sortie d'air (44) est disposée de façon concentrique par rapport à l'ouverture de sortie (38) et entoure celle-ci entièrement.

3. Dispositif selon la revendication 1 ou 2, dans lequel le dispositif de broyage (22) présente (i) un moteur (34), (ii) un aimant (30) entraîné en rotation par le moteur (34) ainsi que (iii) une bille (28) en matériau magnétisable, dans lequel la bille (28) est disposée dans le récipient de réception (14) et le moteur (34) ainsi que l'aimant (30) sont disposés à l'extérieur du récipient de réception (14).

4. Dispositif selon la revendication 3, dans lequel le dispositif de broyage (22) présente exactement une bille (28) en matériau magnétisable.

5. Dispositif selon la revendication 3 ou 4, dans lequel le récipient de réception (14) comporte une face de fond ronde fermée (18).

6. Dispositif selon la revendication 5, dans lequel le diamètre de la face de fond (18) correspond à au moins 5 fois, de préférence à au moins 10 fois le diamètre de la bille (28).

7. Dispositif selon l'une quelconque des revendications 3 à 6, dans lequel le moteur (34) présente un arbre de moteur (32), sur lequel l'aimant (30) est disposé de façon excentrique.

8. Dispositif selon la revendication 7, dans lequel le dispositif de broyage (22) présente un contrepoids (52), qui correspond environ au poids de l'aimant (30), donc entre 90 % et 110 % du poids de l'aimant (30), et est disposé de façon excentrique sur l'arbre de moteur (32), sur un côté opposé à l'aimant (30).

9. Dispositif selon l'une quelconque des revendications 3 à 8, dans lequel le dispositif (10) présente une unité de commande (48) pour la commande du moteur (34), dans lequel l'unité de commande (48) est conçue pour faire fonctionner le moteur (34) avec une première vitesse de rotation nominale, dans lequel la première vitesse de rotation nominale est comprise entre 1500 et 2500 tours par minute.

10. Dispositif selon la revendication 9, dans lequel l'unité de commande (48) est conçue pour changer la vitesse de rotation nominale à des intervalles de temps réguliers de façon temporaire en une seconde vitesse de rotation nominale et pour revenir à chaque fois ensuite de nouveau à la première vitesse de rotation nominale, dans lequel la seconde vitesse de rotation nominale est plus élevée que la première vitesse de rotation nominale.

11. Dispositif selon la revendication 9 ou 10, le dispositif (10) présentant un dispositif d'entrée (50) pour la définition de la première vitesse de rotation nominale par l'utilisateur.

12. Dispositif selon la revendication 9 ou 10, le dispositif (10) présentant un capteur de température pour produire un signal de température et un hygromètre pour produire un signal d'humidité, dans lequel l'unité de commande (48) est conçue pour réguler la première vitesse de rotation nominale en fonction du signal de température et du signal d'humidité.

13. Dispositif selon l'une quelconque des revendications 1 à 12, dans lequel la colonne montante (24) est incurvée.

14. Dispositif selon l'une quelconque des revendications 1 à 13, dans lequel le récipient de réception (14) est disposé de façon séparable sur le boîtier (12).

15. Dispositif selon l'une quelconque des revendications 1 à 14, le dispositif (10) présentant un éclairage pour l'éclairage de l'intérieur du récipient de réception (14) et/ou pour l'éclairage de l'ouverture de sortie (38).
